# EUROPEAN PATENT APPLICATION

(11) **EP 4 130 023 A1**
(43) Date of publication of application: **08.02.2023**
(21) Application number: 21776970.2
(22) Date of filing: 23.03.2021
(51) Int. Cl.: C07K 14/36, C12M 1/34, C12M 1/40, C12N 5/10, C12N 1/15, C12N 1/19, C12N 1/21, C12N 15/31, C12N 15/53, C12N 15/63, C12Q 1/00, C12Q 1/26, C12N 9/06, G01N 33/68, C12P 21/02

(54) **L-GLUTAMIC ACID OXIDAZE MUTANT**

(30) Priority: 24.03.2020 JP 2020052809; 20.08.2020 JP 2020139357
(71) Applicant: AJINOMOTO CO., INC., Chuo-ku Tokyo 104-8315 (JP)
(72) Inventor: TAKAHASHI, Kazutoshi, Kawasaki-shi, Kanagawa 210-8681 (JP); YAMAGUCHI, Hiroki, Kawasaki-shi, Kanagawa 210-8681 (JP); TAGAMI, Uno, Kawasaki-shi, Kanagawa 210-8681 (JP)
(74) Representative: Strehl Schübel-Hopf & Partner
(86) International application number: PCT/JP2021/011898
(87) International publication number: WO 2021/193598

(57) **Abstract**

The present invention provides an alternative L-glutamate oxidase that allows for measurement of L-glutamate. More specifically, the present invention provides the following L-glutamate oxidase mutant (a) or (b) and the like:
(a) an L-glutamate oxidase mutant including an amino acid sequence that has 90% or more identity to an amino acid sequence of SEQ ID NO: 3 and exhibits an activity of oxidizing L-glutamate,
except an L-glutamate oxidase including an amino acid sequence of SEQ ID NO: 1; or
(b) an L-glutamate oxidase mutant comprising a peptide linker consisting of 1 to 20 amino acid residues which is inserted into one or more sites selected from the group consisting of (1) a site in a region proximity to a boundary between α1 and α2 regions, (2) a site in a region proximity to a boundary between α2 and γ regions and (3) a site in a region proximity to a boundary between γ and β regions in the L-glutamate oxidase mutant (a), and having the activity of oxidizing L-glutamate.

## Description

### FIELD

The present invention relates to L-glutamate oxidase mutants and the like.

### BACKGROUND

L-glutamate oxidase (GluOX) is an enzyme that catalyzes the following reaction (EC1.4.3.11).

L-glutamate + O₂ + H₂O → 2-oxoglutarate + H₂O₂+ NH₃

Previously, GluOX was known to exhibit an activity as a heteromultimer (α₂β₂γ₂) with different subunits, by analysis based on isolation and purification of protein, but a gene encoding GluOX was not identified. According to a later report, the GluOX gene encodes an immature protein (molecular weight of approximately 70 kDa) containing an αβγ-containing chain (single chain), and GluOX retains the activity even as a homodimer ((αβγ-containing single chain)₂) with two subunits each containing the αβγ-containing single chain (Patent Literature 1). As a result of subsequent detailed analysis, it is now believed that a natural form of GluOX is not only cleaved by a protease into three segments αβγ, but also cleaved by the protease within an α region to form a unique hetero-octameric 3D structure (α1₂α2₂β₂γ₂) containing two α fragments (referred to as α1 and α2 in this description as required), a β unit, and a γ unit so as to exhibit its activity (Non Patent Literature 1).

L-glutamate is known as a major umami component in foods and is also an important component present in biological samples such as blood. L-glutamate can be easily measured using GluOX. Therefore, GluOX has been used for measurement of L-glutamate in samples.

### CITATION LIST

### PATENT LITERATURE

Patent Literature 1: WO2001/079503 Non Patent Literature
Non Patent Literature 1: Arima J et al., FEES J. 2009 Jul; 276(14): 3894-903.

### SUMMARY

### TECHNICAL PROBLEM

The purpose of the present invention is to provide an L-glutamate oxidase that enables the measurement of L-glutamate.

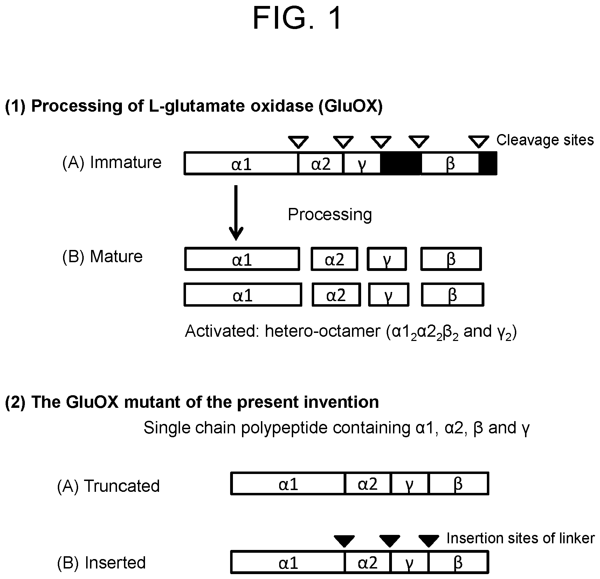

The inventors of the present invention unexpectedly found that a higher activity of oxidizing L-glutamate can be achieved by use of a single-chain polypeptide (polypeptide consisting of the amino acid sequence of SEQ ID NO: 3) lacking both a processing elimination region (a region consisting of 467th to 506th amino acid residues in the amino acid sequence of SEQ ID NO: 1) between γ and β regions and a processing elimination region (a region consisting of 670th to 687th amino acid residues in the amino acid sequence of SEQ ID NO: 1) in a downstream of the β region in a αβγ-containing single-chain GluOX (the amino acid sequence of SEQ ID NO: 1) described in Patent Literature 1, relative to an αβγ-containing single-chain GluOX described in Patent Literature 1 (FIG. 1 and Examples).

The inventors of the present invention also found that a higher activity of oxidizing L-glutamate can be achieved by use of a single-chain polypeptide with a peptide linker inserted in at least one or more boundary proximity regions: a region in proximity to a boundary between α1 and α2 regions, a region in proximity to a boundary between α2 and γ regions, and a region in proximity to a boundary between γ and β regions (in particular, the region in proximity to the boundary between α1 and α2 regions, and the region in proximity to the boundary between α2 and γ regions) in the above single chain polypeptide, relative to the above immature protein (FIG. 1, Example).

Furthermore, the inventors of the present invention succeeded in finding several mutants capable of exhibiting higher activities of oxidizing L-glutamate relative to that of the αβγ-containing single-chain GluOX described in Patent Literature 1, and completed the present invention.

### SOLUTION TO PROBLEM

Accordingly, the present invention is as follows.
[1] An L-glutamate oxidase mutant of (a) or (b):
   (a) an L-glutamate oxidase mutant comprising an amino acid sequence that has 90% or more identity to the amino acid sequence of SEQ ID NO: 3 and exhibits an activity of oxidizing L-glutamate except for an L-glutamate oxidase comprising the amino acid sequence of SEQ ID NO: 1; or
   (b) an L-glutamate oxidase mutant comprising a peptide linker consisting of 1 to 20 amino acid residues which is inserted into one or more sites selected from the group consisting of (1) a site in a region in proximity to a boundary between α1 and α2 regions, (2) a site in a region in proximity to a boundary between α2 and γ regions and (3) a site in a region in proximity to a boundary between γ and β regions in the L-glutamate oxidase mutant (a), and the L-glutamate oxidase mutant having the activity of oxidizing L-glutamate,
      wherein the site in the region in proximity to the boundary between α1 and α2 regions is a site in a region consisting of 349th to 363rd amino acid residues of SEQ ID NO: 3,
      the site in the region in proximity to the boundary between α2 and γ regions is a site in a region consisting of 372nd to 377th amino acid residues of SEQ ID NO: 3, and
      the site in the region in proximity to the boundary between γ and β regions is a site in a region consisting of 466th to 469th amino acid residues of SEQ ID NO: 3.
[2] The L-glutamate oxidase mutant according to [1], wherein the L-glutamate oxidase mutant is a mutant of an L-glutamate oxidase derived from a microorganism belonging to the genus *Streptomyces.*
[3] The L-glutamate oxidase mutant according to [2], wherein the microorganism belonging to the genus *Streptomyces* is *Streptomyces sp.* X-119-6.
[4] The L-glutamate oxidase mutant according to any of [1] to [3], wherein the peptide linker is inserted into either one or both of (1) the site in the region in proximity to the boundary between α1 and α2 regions, or (2) the site in the region in proximity to the boundary between α2 and γ regions.
[5] The L-glutamate oxidase mutant according to any of [1] to [4],
   wherein the site in the region in proximity to the boundary between α1 and α2 regions is a site between 356th and 357th amino acid residues in SEQ ID NO: 3,
   the site in the region in proximity to the boundary between α2 and γ regions is a site between 376th and 377th amino acid residues in SEQ ID NO: 3, or
   the site in the region in proximity to the boundary between γ and β regions is a site between 466th and 467th amino acid residues in SEQ ID NO: 3.
[6] The L-glutamate oxidase mutant according to any of [1] to [5], comprising a mutation or mutations of one or more amino acid residues selected from the group consisting of A106, C210, Q235, D236, D237, P244, T311, W313, Q333, I334, M336, Q338, R339, T416, A438, K441, Y455, Q456, Q457, L505, P558, C561 and P569 in SEQ ID NO: 3.
[7] The L-glutamate oxidase mutant according to any of [1] to [6], comprising a mutation or mutations of one or more amino acid residues selected from the group consisting of A106S, C210S, Q235E, D236E, D237E, P244H, T311S, W313F, Q333E, I334V, I334L, M336L, Q338E, R339K, T416S, A438P, K441E, Y455F, Q456R, Q457E, Q457K, L505I, P558A, C561S and P569A in SEQ ID NO: 3.
[8] A method of analyzing L-glutamate, the method comprising measuring L-glutamate contained in a test sample using the L-glutamate oxidase mutant according to any of [1] to [7] .
[9] The method according to [8], wherein the L-glutamate is measured using N-ethyl-N-(2-hydroxy-3-sulfopropyl)-3-methoxyaniline (TOOS) and 4-aminoantipyrine and a peroxidase in addition to the L-glutamate oxidase mutant according to any of [1] to [7].
[10] A method for producing 2-oxoglutaric acid, the method comprising producing 2-oxoglutaric acid from L-glutamate in presence of the L-glutamate oxidase mutant according to any of [1] to [7].
[11] A polynucleotide encoding the L-glutamate oxidase mutant according to any of [1] to [7].
[12] An expression vector comprising the polynucleotide according to [11].
[13] A transformed microorganism comprising an expression unit containing a polynucleotide encoding the L-glutamate oxidase mutant according to any of [1] to [7] and a promoter operably linked to the polynucleotide.
[14] A method of producing the L-glutamate oxidase mutant according to any of [1] to [7], comprising producing the L-glutamate oxidase mutant using the transformed microorganism according to [13].
[15] An L-glutamate detection reagent or kit comprising the L-glutamate oxidase mutant according to any of [1] to [7].
[16] The L-glutamate detection reagent or kit according to [15], further comprising one or more selected from the group consisting of a buffer solution or buffer salt for reaction, a hydrogen peroxide detection reagent, an ammonia detection reagent, and a 2-oxoglutaric acid detection reagent.
[17] A detection system for analysis of L-glutamate, the detection system comprising:
   (a) a device; and
   (b) the L-glutamate oxidase mutant according to any of [1] to [7] .
[18] The detection system for analysis of L-glutamate according to [17], the system further comprising (c) one or more selected from the group consisting of a buffer solution or buffer salt for reaction, a hydrogen peroxide detection reagent, an ammonia detection reagent, and a 2-oxoglutaric acid detection reagent.
[19] An enzyme sensor for analysis of L-glutamate, the enzyme sensor comprising:
   (a) an electrode for detection; and
   (b) the L-glutamate oxidase mutant according to any of [1] to [7] that is immobilized or retained on the electrode for detection.

### EFFECTS OF INVENTION

The L-glutamate oxidase mutant of the present invention has a high activity against L-glutamate and is therefore useful for fast and sensitive measurement of L-glutamate and/or production of 2-oxoglutarate. The L-glutamate oxidase mutant of the present invention is easily prepared because no protease treatment is required and no subsequent purification process is required for the protease treated product. An analytical method of the present invention is useful in a wide range of fields such as biological research, health nutrition, medication, and food production.

### BRIEF DESCRIPTION OF DRAWINGS

FIG. 1 represents relationship between processing of L-glutamate oxidase (GluOX) and L-glutamate oxidase mutant of the present invention. The GluOX gene naturally expresses a single-chain polypeptide that encodes α regions (α1 region and α2 region), β region and γ region. In nature, this single-chain polypeptide is known to be cleaved by processing with a protease expressed by a microorganism that naturally produces this single-chain polypeptide (microorganism belonging to the genus *Streptomyces*) to form a hetero-octamer (α1₂α2₂β₂γ₂) . The GluOX mutant of the present invention refers to (A) a truncated single-chain polypeptide lacking both a processing elimination region between γ and β regions and a processing elimination region in a downstream of the β region in the single-chain polypeptide, and (B) an inserted single-chain polypeptide with a peptide linker inserted in one or more sites selected from the group consisting of (1) a site in a region in proximity to a boundary between α1 and α2 regions, (2) a site in a region in proximity to a boundary between α2 and γ regions and (3) a site in a region in proximity to a boundary between γ and β regions in the truncated single-chain polypeptide (A).

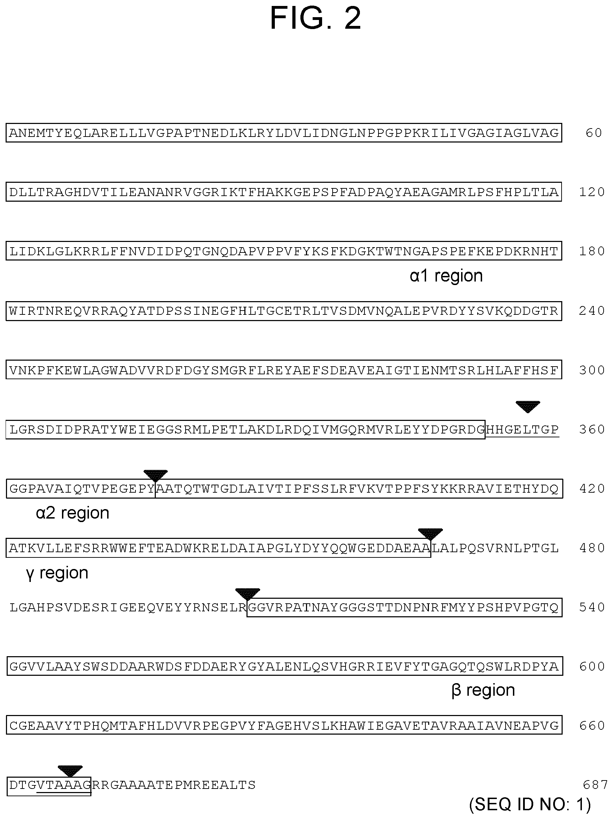

FIG. 2 represents the amino acid sequence of the wild-type GluOX of *Streptomyces sp.* X-119-6 (SEQ ID NO: 1). The amino acid sequence of this wild-type GluOX (SEQ ID NO: 1) is encoded by the nucleotide sequence of SEQ ID NO: 2. Each of regions in the amino acid sequence of SEQ ID NO: 1 corresponds to the following. α-region: a region consisting of 1st to 376th amino acid residues (α1 region: a region consisting of 1st to 352nd amino acid residues; α2 region: a region consisting of 361st to 376th amino acid residues); γ-region: a region consisting of 377th to 466th amino acid residues; and β-region: a region consisting of 507th to 669th amino acid residues. Protease cleavage sites (indicated by black triangles) in the amino acid sequence of SEQ ID NO: 1 correspond to the following. Protease cleavage region in α region: a region between 353rd to 360th amino acid residues (underlined); a boundary site between α and γ regions: a site between 376th and 377th amino acid residues; a downstream site of γ region: a site between 466th and 467th amino acid residues; an upstream site of β region between 506th and 507th amino acid residues; a downstream region of β region: a region between 664th and 669th amino acid residues (underlined). Preferred positions at which point mutation can be introduced for the L-glutamate oxidase mutants are A106, C210, Q235, D236, D237, P244, T311, W313, Q333, I334, M336, Q338, R339, T416, A438, K441, Y455, Q456, Q457, L545, L598, C601 and P609.

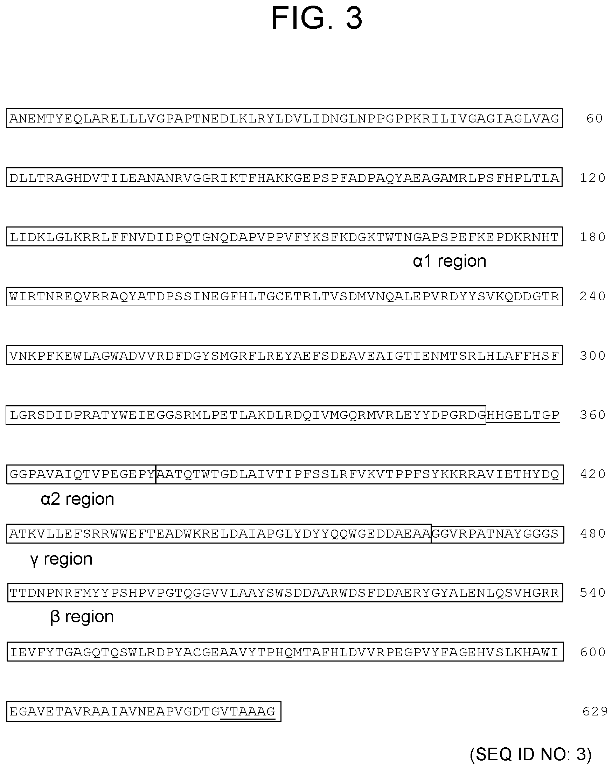

FIG. 3 represents a truncated single-chain polypeptide lacking both a processing elimination region between γ and β regions (a region consisting of 467th to 506th amino acid residues in the amino acid sequence of SEQ ID NO: 1) and a processing elimination region in a downstream of the β region (a region consisting of 670th to 687th amino acid residues in the amino acid sequence of SEQ ID NO: 1) in the amino acid sequence of the wild-type GluOX of *Streptomyces sp.* X-119-6 (SEQ ID NO: 1) (polypeptide consisting of the amino acid sequence of SEQ ID NO: 3). Each of regions in the amino acid sequence of SEQ ID NO: 3 corresponds to the following. α-region: a region consisting of 1st to 376th amino acid residues (α1 region: a region consisting of 1st to 352nd amino acid residues; α2 region: a region consisting of 361st to 376th amino acid residues); γ-region: a region consisting of 377th to 466th amino acid residues; and β-region: a region consisting of 467th to 629th amino acid residues. Each of proximity regions in the amino acid sequence of SEQ ID NO: 3 corresponds to the followings. A region in proximity to a boundary between α1 and α2 regions: a region consisting of 349th to 363rd amino acid residues; a region in proximity to a boundary between α2 and γ regions: a region consisting of 372nd to 377th amino acid residues; and a region in proximity to a boundary between γ and β regions: a region consisting of 466th to 469th amino acid residues. The underlined areas correspond to the protease cleavage regions within the α region described in FIG. 2; and the downstream region of the β region. Preferred positions at which point mutation can be introduced for the L-glutamate oxidase mutants are A106, C210, Q235, D236, D237, P244, T311, W313, Q333, I334, M336, Q338, R339, T416, A438, K441, Y455, Q456, Q457, L505 (corresponding to L545 in SEQ ID NO: 1), P558 (corresponding to L598 in SEQ ID NO: 1), C561 (corresponding to C601 in SEQ ID NO: 1), and P569 (corresponding to P609 in SEQ ID NO: 1).

### EMBODIMENTS FOR CARRYING OUT THE INVENTION

The present invention provides an L-glutamate oxidase mutant of (a) or (b):
(a) an L-glutamate oxidase mutant comprising an amino acid sequence that has 90% or more identity to the amino acid sequence of SEQ ID NO: 3 and exhibits an activity of oxidizing L-glutamate,
   except an L-glutamate oxidase comprising the amino acid sequence of SEQ ID NO: 1; or
(b) an L-glutamate oxidase mutant comprising a peptide linker consisting of 1 to 20 amino acid residues which is inserted into one or more sites selected from the group consisting of (1) a site in a region in proximity to a boundary between α1 and α2 regions, (2) a site in a region in proximity to a boundary between α2 and γ regions and (3) a site in a region in proximity to a boundary between γ and β regions in the L-glutamate oxidase mutant (a), and having the activity of oxidizing L-glutamate,
   wherein the site in the region in proximity to the boundary between α1 and α2 regions is a site in a region consisting of 349th to 363rd amino acid residues of SEQ ID NO: 3,
   the site in the region in proximity to the boundary between α2 and γ regions is a site in a region consisting of 372nd to 377th amino acid residues of SEQ ID NO: 3, and
   the site in the region in proximity to the boundary between γ and β regions is a site in a region consisting of 466th to 469th amino acid residues of SEQ ID NO: 3.

The L-glutamate oxidase mutant (a) corresponds to a truncated single chain polypeptide lacking both a processing elimination region between γ and β regions (a region consisting of 467th to 506th amino acid residues in the amino acid sequence of SEQ ID NO: 1) and a processing elimination region in a downstream of the β region (a region consisting of 670th to 687th amino acid residues in the amino acid sequence of SEQ ID NO: 1) in the single-chain polypeptide naturally expressed by a GluOX gene (the amino acid sequence of SEQ ID NO: 1) (polypeptide of the amino acid sequence of SEQ ID NO: 3). Therefore, the L-glutamate oxidase mutant (a) does not contain the regions consisting of 467th to 506th and 670th to 687th amino acid residues in the amino acid sequence of SEQ ID NO: 1.

A percentage of the amino acid sequence identity to the amino acid sequence of SEQ ID NO: 3 in the L-glutamate oxidase mutant (a) may be preferably 92% or more, more preferably 95% or more, still more preferably 97% or more, and most preferably 98% or more or 99% or more. The percentage of the identity of the amino acid sequence can be calculated with GENETYX Ver. 13.1.1 software available from GENETYX CORPORATION by performing Muscle alignment, ClustalW alignment or Multiple sequence alignment using a full length of a polypeptide part encoded in ORF and then calculating while gaps are taken into account to obtain a value for use.

In the L-glutamate oxidase mutant (b), the region in which the peptide linker is inserted refers to one or more (e.g., one, two or three) sites selected from the group consisting of (1) the site in the region in proximity to the boundary between the α1 and α2 regions (the region consisting of 349th to 363rd amino acid residues in SEQ ID NO: 3), (2) the site in the region in proximity to the boundary between the α2 and γ regions (the region consisting of 372nd to 377th amino acid residues in SEQ ID NO: 3), and (3) the site in the region in proximity to the boundary between the γ and β regions (the region consisting of 466th to 469th amino acid residues in SEQ ID NO: 3). Preferably, the region in which the peptide linker is inserted may be either one or both of (1) the site in the region in proximity to the boundary between the α1 and α2 regions, and (2) the site in the region in proximity to the boundary between the α2 and γ regions.

The site in the region in proximity to the boundary between the α1 and α2 regions is preferably the site in the boundary region between the α1 and α2 regions (the region consisting of 349th to 363rd amino acid residues in SEQ ID NO: 3), and more preferably the site between 356th to 357th amino acid residues in SEQ ID NO: 3.

The site in the region in proximity to the boundary between the α2 and γ regions is the site in the region in proximity to the boundary between the α2 and γ regions (the region consisting of 372nd to 377th amino acid residues in SEQ ID NO: 3), preferably the site between 376th to 377th amino acid residues in SEQ ID NO: 3.

The site in the region in proximity to the boundary between the γ and β regions is the site in the region in proximity to the boundary between the γ and β regions (the region consisting of 466th to 469th amino acid residues in SEQ ID NO: 3), preferably the site between 466th and 467th amino acid residues in SEQ ID NO: 3.

A peptide linker is a peptide linker consisting of 1 to 20 amino acid residues. The peptide linker may consist of an amino acid sequence that is different from a partial amino acid sequence of the L-glutamate oxidase. The peptide linker may consist of two or more, three or more, four or more, or five or more amino acid residues. The peptide linker may consist of 18 or fewer, 16 or fewer, 14 or fewer, 12 or fewer, or 10 or fewer amino acid residues. More specifically, the peptide linker may consist of 2 to 18, 3 to 16, 4 to 14, 5 to 12, or 5 to 10 amino acid residues.

The types of amino acid residues constituting the peptide linker refer to glycine (G) residue and residues of the naturally-occurring L-α-amino acid constituting general proteins. Specific examples of the residues of such a naturally-occurring L-α-amino acid include L-alanine (A), L-asparagine (N), L-cysteine (C), L-glutamine (Q), L-isoleucine (I), L-leucine (L), L-methionine (M), L-phenylalanine (F), L-proline (P), L-serine (S), L-threonine (T), L-tryptophan (W), L-tyrosine (Y), L-valine (V), L-aspartic acid (D), L-glutamic acid (E), L-arginine (R), L-histidine (H) and L-lysine (K). Preferably, the type of amino acid residue constituting the peptide linker refers to amino acid residues capable of easily constituting a peptide linker with high flexibility. Examples of the amino acid residues capable of easily constituting the peptide linker with high flexibility include glycine (G), L-alanine (A), L-serine (S), and L-threonine (T).

Preferably, the peptide linker is GGGGS (SEQ ID NO: 4) or a repeated sequence thereof. The number of repetitions in the repeated sequence is two to four, but preferably two or three, and more preferably two.

The activity of oxidizing L-glutamate is an activity that catalyzes the following reaction.

L-glutamate + O₂ + H₂O → 2-oxoglutarate + H₂O₂+ NH₃

The L-glutamate-oxidizing activity of the L-glutamate oxidase mutant of the present invention is not particularly limited as long as it is comparable to or higher than that of the L-glutamate oxidase consisting of the amino acid sequence of SEQ ID NO: 1, but preferably a 1.1-fold or higher L-glutamate-oxidizing activity (more preferably a 1.2-fold or higher L-glutamate-oxidizing activity) compared to that of the L-glutamate oxidase consisting of the amino acid sequence of SEQ ID NO: 1. Such an activity of oxidizing L-glutamate can be measured by utilizing the oxidation reaction of L-glutamate (10 mM) and its conjugation reaction, as described in Examples.

(Oxidation reaction of L-glutamate) A reaction catalyzed by L-glutamate oxidase

L-glutamate + O₂ + H₂O → 2-oxoglutarate + H₂O₂+ NH₃

(Conjugation reaction) Reaction catalyzed by peroxidase H₂O₂+ TOOS + 4-AA → dye compound (absorbance: approx. 555 nm) TOOS: N-ethyl-N-(2-hydroxy-3-sulfopropyl)-3-methoxyaniline 4-AA: 4-Aminoantipyrine

As well, the L-glutamate-oxidizing activity of the L-glutamate oxidase mutants of the present invention is not particularly limited as long as they exhibit a higher L-glutamate-oxidizing activity than an αβγ-containing single-chain L-glutamate oxidase described in Patent Literature 1. Preferably, the L-glutamate-oxidizing activity of the L-glutamate oxidase mutant of the present invention may be comparable to or higher than that of the L-glutamate oxidase consisting of the amino acid sequence of SEQ ID NO: 3. The L-glutamate-oxidizing activity of the L-glutamate oxidase mutant of the present invention may be more preferably a 1.1-fold or more L-glutamate-oxidizing activity, even more preferably a 1.2-fold or more L-glutamate-oxidizing activity, and particularly preferably a 1.3-fold or more, 1.4-fold or more, 1.5-fold or more, 1.6-fold or more, 1.7-fold or more, or 1.8-fold or more L-glutamate-oxidizing activity higher than that of an αβγ-containing single-chain L-glutamate oxidase described in Patent Literature 1 or the L-glutamate oxidase consisting of the amino acid sequence of SEQ ID NO: 3 (preferably the L-glutamate oxidase consisting of the amino acid sequence of SEQ ID NO: 3). Such an oxidizing activity of L-glutamate can be measured by utilizing the oxidation reaction of L-glutamate (10 mM) and its conjugation reaction, as described above.

The L-glutamate oxidase mutant of the present invention may have one or more mutation or mutations capable of enhancing the oxidizing activity of the L-glutamate oxidase mutant. Examples of such mutations include mutations of one or more (e.g., one, two, or three) amino acid residues selected from the group consisting of A106, C210, Q235, D236, D237, P244, T311, W313, Q333, I334, M336, Q338, R339, T416, A438, K441, Y455, Q456, Q457, L505, P558, C561 and P569 in SEQ ID NO: 3. The amino acid residues obtained after the mutation at these sites are the desired naturally-occurring L-α-amino acid residues that differ from the amino acid residues prior to the mutation. Such a desired naturally-occurring L-alpha-amino acid residue is L-alanine (A), L-asparagine (N), L-cysteine (C), L-glutamine (Q), L-isoleucine (I), L-leucine (L), L-methionine (M), L-phenylalanine (F), L-proline (P), L-serine (S), L-threonine (T), L-tryptophan (W), L-tyrosine (Y), L-valine (V), L-aspartic acid (D), L-glutamic acid (E), L-arginine (R), L-histidine (H), L-lysine (K) or L-glutamic acid (G) residue. When the L-glutamate oxidase mutants of the present invention have two or more mutations, such mutations may be two or more mutations that include a combination of D236 with another mutation (e.g., D236 and Q338, D236 and R339, D236 and T416), a combination of T311 with another mutation (e.g., T311 and D236, T311 and Q457), and a combination of Q457 with another mutation (e.g., Q457 and D236, Q457 and T311, Q457 and Q338, Q457 and R339) .

Preferably, the one or more mutation or mutations capable of enhancing the oxidizing activity of the L-glutamate oxidase mutant is preferably a mutation or mutations of one or more (e.g., 1, 2, or 3) amino acid residues selected from the group consisting of A106S, C210S, Q235E, D236E, D237E, P244H, T311S, W313F, Q333E, I334V, I334L, M336L, Q338E, R339K, T416S, A438P, K441E, Y455F, Q456R, Q457E, Q457K, L505I, P558A, C561S and P569A in SEQ ID NO: 3. When the L-glutamate oxidase mutants of the present invention have two or more mutations, such mutations may be two or more mutations that include a combination of D236E with another mutation (e.g., D236E and Q338E, D236E and R339K, D236E and T416S), a combination of T311S with another mutation (e.g., T311S and D236E, T311S and Q457E), and a combination of Q457E with another mutation (e.g., Q457E and D236E, Q457E and T311S, Q457E and Q338E, Q457E and R339K) .

As the L-glutamate oxidase from which the L-glutamate oxidase mutant of the present invention is derived, enzymes from any organism (e.g., microorganisms, animals, and plants) can be used, but enzymes from microorganisms belonging to the the genus *Streptomyces* are preferred. As the microorganism belonging to the genus *Streptomyces, Streptomyces sp.* X-119-6 is preferred.

The L-glutamate oxidase mutant of the present invention may further contain another peptide component (e.g., a tag moiety) at a C-terminus or an N-terminus thereof. Examples of the other peptide component containable in the L-glutamate oxidase mutant of the present invention include peptide components facilitating purification of a targeted protein (e.g., tag moieties such as histidine tag and strep-tag II; and proteins such as glutathione-S-transferase and maltose-binding protein commonly used for purification of the targeted protein), peptide components (e.g., Nus-tag) enhancing solubility of the targeted protein, peptide components acting as a chaperon (e.g., trigger factor), and peptide components acting as linkers linking the L-glutamate oxidase mutant to either one or both of a protein with another function and a domain thereof.

The modified enzyme of the present invention can be prepared using a transformed microorganism of the present invention expressing the L-glutamate oxidase mutant of the present invention or cell-free system. The transformed microorganism of the present invention can be prepared by preparing an expression vector of the present invention and then introducing this expression vector into a host, for example.

The expression vector of the present invention contains a polynucleotide (e.g., DNA or RNA) of the present invention encoding the L-glutamate oxidase mutant of the present invention. In addition to the polynucleotide of the invention, the expression vector of the invention may further contain a region such as regions encoding promoter, terminator and regions encoding resistant genes against chemicals (e.g., tetracycline, ampicillin, kanamycin, hygromycin and phosphinothricin). The expression vector of the present invention may be a plasmid or an integrative vector. The expression vector of the present invention may be a viral vector or vectors for cell-free system, as well. The expression vector of the present invention may further include a polynucleotide(s) which encode(s) another peptide component(s) addable to the L-glutamate oxidase mutant of the present invention, at a 3' or 5' terminus side of the polynucleotide of the present invention. Examples of the polynucleotide encoding (an)other peptide component(s) include polynucleotides encoding peptide components that facilitate the purification of the target protein as described above, polynucleotides encoding peptide components that improve the solubility of the target protein as described above, polynucleotides encoding peptide components that act as chaperones, and polynucleotides encoding peptide components that act as linkers linking the L-glutamate oxidase mutant to either one or both of the protein with another function and the domain thereof. A variety of expression vectors that contain polynucleotides encoding other peptide components can be used. Therefore, such expression vectors may be used to prepare the expression vectors of the present invention. Examples of the expression vectors (e.g., pET-15b, pET-51b, pET-41a, and pMAL-p5G) with polynucleotides encoding peptide components that facilitate the purification of the target protein include expression vectors (e.g., pET-50b) with polynucleotides encoding peptide components that improve the solubility of the target protein, expression vectors (e.g., pCold TF) with polynucleotides encoding peptide components that act as chaperones, and expression vectors with polynucleotides encoding peptide components that act as linkers linking the L-glutamate oxidase mutant to either one or both of the protein with another function and the domain thereof. In order to allow for cleavage of the attached (an)other peptide component(s) from the L-glutamate oxidase mutant of the present invention after the protein expression, the expression vector of the present invention may include a region encoding a cleavage site to be cleaved by a protease between the polynucleotide encoding the L-glutamate oxidase mutant of the present invention and the polynucleotide encoding (an)other peptide component(s).

Various prokaryotic cells including cells from bacteria belonging to genera Escherichia (e.g., Escherichia coli), Corynebacterium (e.g., Corynebacterium glutamicum) and Bacillus (e.g., Bacillus subtilis), and various eukaryotic cells including cells from fungi belonging to genera Saccharomyces (e.g., Saccharomyces cerevisiae), Pichia (e.g., Pichia stipitis) and Aspergillus (e.g., Aspergillus oryzae) can be used as the host for expressing the modified enzyme of the present invention. In addition, microorganisms (e.g., microorganisms belonging to the genus *Streptomyces*) that do not express proteases capable of cleaving L-glutamate oxidase mutants may be suitably used as hosts for expressing the L-glutamate oxidase mutant of the present invention. A strain in which a certain gene has been deleted may be used as the host. Examples of the transformed microorganism include transformed microorganisms having the vector in its cytoplasm and transformed microorganisms with a targeted gene is integrated into its genome.

The transformed microorganism of the present invention can be cultured in a medium having a composition described later using a given culture apparatus (e.g., test tube, flask, and jar fermenter). A culture condition can appropriately be determined. Specifically, a culture temperature may be 10°C to 37°C, a pH value may be 6.5 to 7.5, and a culture period may be 1 to 100 hours. A cultivation may also be carried out with managing a dissolved oxygen concentration. In this case, the dissolved oxygen concentration (DO value) may be used as an indicator for control. A ventilation/stirring condition can be controlled so that a relative dissolved oxygen concentration, a DO value be not below 1 to 10% for example, and preferably not below 3 to 8% when an oxygen concentration in air is 21%. The cultivation may be a batch cultivation or a fed-batch cultivation. In the case of the fed-batch cultivation, the cultivation can also be continued by sequentially adding continuously or discontinuously a solution as a sugar source and a solution containing phosphate to the culture medium.

The host to be transformed is as described above, and describing Escherichia coli in detail, the host can be selected from Escherichia coli K12 subspecies Escherichia coli JM109 strain, DH5α strain, HB101 strain, BL21 (DE3) strain, and the like. Methods of performing the transformation and methods of selecting the transformant have also been described in Molecular Cloning: A Laboratory Manual, 3rd edition, Cold Spring Harbor press (2001/01/15), and the like. Hereinafter, a method of making transformed Escherichia coli and producing a predetermined enzyme using this will be described specifically by way of example only.

A promoter used for producing a foreign protein in E. coli can generally be used as a promoter for expressing the polynucleotide of the present invention. Examples thereof may include potent promoters such as a PhoA, a PhoC, a T7 promoter, a lac promoter, a trp promoter, a trc promoter, a tac promoter, PR and PL promoters of lambda phage, and a T5 promoter, and the PhoA, the PhoC and the lac promoters are preferred. For example, pUC (e.g., pUC19 or pUC18), pSTV, pBR (e.g., pBR322), pHSG (e.g., pHSG299, pHSG298, pHSG399 or pHSG398), RSF (e.g., RSF1010), pACYC (e.g., pACYC177 or pACYC184), pMW (e.g., pMW119, pMW118, pMW219 or pMW218), pQE (e.g., pQE30) and derivatives thereof may be used as a vector. A vector from phage DNA may also be utilized as the other vector. Furthermore, an expression vector that includes a promoter and can express an inserted DNA sequence may also be used. Preferably, the vector may be pUC, pSTV, or pMW.

Also, a terminator that is a transcription terminating sequence may be ligated downstream of the polynucleotide of the present invention. Examples of such a terminator may include a T7 terminator, an fd phage terminator, a T4 terminator, a terminator of a tetracycline resistant gene, and a terminator of Escherichia coli trpA gene.

The vector for introducing the polynucleotide of the present invention into Escherichia coli is preferably a so-called multicopy type, and examples thereof may include plasmids which have a replication origin from ColE1, such as pUC-based plasmids, pBR322-based plasmids and derivatives thereof. Here the "derivative" means those in which the modification has been given to the plasmid by substitution, deletion, insertion and/or addition of base(s).

In order to select the transformed microorganism, the vector preferably has a marker such as an ampicillin resistant gene. Expression vectors having a potent promoter are commercially available as such a plasmid (e.g., pUC-based (supplied from Takara Bio Inc.), pPROK-based (supplied from Clontech), pKK233-2-based (supplied from Clontech)).

The L-glutamate oxidase mutant of the present invention can be obtained by transforming Escherichia coli using the resulting expression vector of the present invention and culturing this Escherichia coli.

Media such as M9/casamino acid medium and LB medium generally used for culturing Escherichia coli may be used as the medium. The medium may contain a predetermined carbon source, nitrogen source, or coenzyme (e.g., pyridoxine hydrochloride). Specifically, peptone, yeast extract, NaCl, glucose, MgSO₄, ammonium sulfate, potassium dihydrogen phosphate, ferric sulfate, manganese sulfate, and the like may be used. A cultivation condition and a production inducing condition are appropriately selected depending on types of a marker and a promoter in a vector and a host and the like to be used.

The L-glutamate oxidase mutant of the present invention can be recovered by the following methods. The L-glutamate oxidase mutant of the present invention can be obtained as a disrupted or lysed product by collecting the transformed microorganism of the present invention and subsequently disrupting (e.g., sonication or homogenization) or lysing (e.g., treatment with lysozyme) the microbial cells. The modified enzyme of the present invention can be obtained by subjecting such a disrupted or lysed product to techniques such as extraction, precipitation, filtration, and column chromatography.

The present invention also provides a method of analyzing L-glutamate. The analysis method of the present invention can include measuring L-glutamate contained in a test sample using the L-glutamate oxidase mutant of the present invention.

The test sample is not particularly limited as long as the sample is suspected of containing L-glutamate, and examples thereof may include biological samples (e.g., blood, urine, saliva, and tear) and food and beverage (e.g., nutrient drinks and amino acid beverages). A concentration of L-glutamate in the test sample may be low (e.g., concentration less than 1 mM, such as 1 µM or more and less than 1 mM) or high (e.g., concentration of 1 mM or more, such as 1 mM or more and less than 1 M).

The analytical method of the present invention is not particularly limited as long as L-glutamate can be measured using the L-glutamate oxidase mutant of the present invention, and may involve detection of the generated 2-oxoglutarate or detection of a side product of NH₃ or H₂O₂ generated together with 2-oxoglutarate. Alternatively, the method may involve detecting a conjugation reaction product that is obtained by conjugation with another reaction. The following conjugation reaction is an example of such a conjugation reaction.

(Oxidation reaction of L-glutamate) Reaction catalyzed by L-glutamate oxidase

L-glutamate + O₂ + H₂O → 2-oxoglutarate + H₂O₂+ NH₃

(Conjugation reaction) Reaction catalyzed by peroxidase H₂O₂+ TOOS + 4-AA → dye compound (absorbance: approx. 555 nm) TOOS and 4-AA are the same as above.

In the case of utilizing the above conjugation reaction, L-glutamate can be measured using TOOS, 4-AA and peroxidase, in addition to the L-glutamate oxidase mutant of the present invention. Specifically, the test sample is mixed with TOOS, 4-AA and peroxidase in an aqueous solution (e.g., buffer). The mixed sample is then subjected to the enzymatic reaction described above. Finally, the absorbance of the produced (about 555 nm) is detected from the resulting dye compound for the measurement of L-glutamate. The measurement can be performed qualitatively or quantitatively. The measurement may be performed according to an endpoint method in which the measurement is performed until all substrates are reacted, or a rate method (initial rate method), for example. Because of a small amount of oxygen required in the oxidation reaction, with an adequate amount of oxygen dissolved in the reaction system, there is generally no need to compulsorily supply oxygen or oxygen-containing gases into the reaction system.

The L-glutamate oxidase mutant of the present invention is not or poorly reacted with amino acids (L-α-amino acid) other than L-glutamate. Therefore, even when not only L-glutamate but also other amino acids are contained in the test sample, an amount of L-glutamate in the test sample can be specifically evaluated by using the L-glutamate oxidase mutant of the present invention.

The use of a hydrogen peroxide electrode with the L-glutamate oxidase mutant of the present invention enables the specific evaluation of the amount of L-glutamate in the test sample.

Furthermore, the present invention includes a kit that is used for the analysis of L-glutamate and includes (A) the L-glutamate oxidase mutant of the present invention. The kit of the present invention can further include at least one of (B) a buffer solution or a buffer salt for reaction, (C) a hydrogen peroxide detection reagent, (D) an ammonia detection reagent, and (E) a 2-oxoglutarate detection reagent.

The buffer solution or the buffer salt for the reaction is used for keeping a pH value in a reaction solution suitable for an objective enzymatic reaction.

(C) The hydrogen peroxide detection reagent is used when hydrogen peroxide is detected by coloration or fluorescence, for example. Examples of a combination of the peroxidase with a chromogenic agent capable of serving as a substrate for the peroxidase specifically include a combination of horseradish peroxidase with 4-aminoantipyrine and phenol, or the like, but the use of the hydrogen peroxide detection reagents is not limited to these combinations.

(D) The ammonia detection reagent can be used in an indophenol method with combination use of phenol and hypochlorite, for example.

(E) An example of 2-Oxoglutarate detection reagent is 2-oxoacid reductase.

The present invention also provides a detection system that is used for the analysis of L-glutamate and includes (a) a device and (b) the L-glutamate oxidase mutant of the present invention.

The L-glutamate oxidase mutant of the present invention may be present as a unit independent of a microdevice suppliable for use in the device, and may be injected, fixed or placed into the device beforehand. Preferably, the L-glutamate oxidase mutant of the present invention is provided while injected, fixed or placed into the device beforehand. The L-glutamate oxidase mutant of the present invention is fixed or placed into the device in a direct or indirect way. For example, a microdevice such as a microfluidic chip equipped with a flow channel can be used as the device.

The detection system for the analysis of L-glutamate may further include one or more components selected from the group consisting of (c) the buffer solution or the buffer salt for reaction, the hydrogen peroxide detection reagent, the ammonia detection reagent and the 2-oxoglutarate detection reagent. The detection system of the present invention for the analysis of L-glutamate may be provided while all of the components (c) are accommodated in the device. Alternatively, it can be provided by accommodating some of the components (c) in the device while not accommodating the remaining component in the device (e.g., accommodating it in a different container). In this case, the component (c) not accommodated in the device may be injected for use into the device during measurement of a target substance.

Examples of the device include: 1) a device (device used for steps of mixing and detecting in different sections) provided with a first section allowing the sample to be mixed with the component (c) for preparation of a mixture solution and a second section allowing the prepared mixture solution to come into contact with the L-glutamate oxidase mutant of the present invention for the purpose of the detection of L-glutamate; 2) a device (device used for the steps of mixing and detecting in a common section) provided with a section allowing the sample to be mixed with the component (c) and the L-glutamate oxidase mutant of the present invention for the purpose of the detection of L-glutamate with use of the L-glutamate oxidase mutant of the present invention; and 3) a device (configured to automatically mix the sample and the like that are flowed through a flow channel by sample injection via an inlet of the device, and automatically detect L-glutamate contained in the resulting mixture solution in a detection section) provided with the flow channel allowing the sample to be mixed with the component (c) (and the L-glutamate oxidase mutant of the present invention, as required) and a section allowing L-glutamate to be detected with use of the L-glutamate oxidase mutant of the present invention. From the viewpoint of automation, the device 3), particularly the device 3) with a microfluidic device, is preferred. In the device (3), the L-glutamate oxidase mutant of the present invention may be provided in the solution flowed through the flow channel or provided while immobilized or retained in the detection section, but preferably provided while immobilized or retained in the detection section.

The present invention also provides an enzyme sensor that is used for the analysis of L-glutamate and includes (a) an electrode for detection and (b) the L-glutamate oxidase mutant of the present invention immobilized or retained on the detection electrode. The L-glutamate oxidase mutant of the present invention is immobilized or retained on the electrode directly or indirectly.

For example, a hydrogen peroxide detection electrode can be used as the above detection electrode. More specific examples of the detection electrode include an enzymatic hydrogen peroxide detection electrode and a diaphragm hydrogen peroxide detection electrode. This case allows hydrogen peroxide to be detected after produced as a result of the oxidation of L-glutamate by the L-glutamate oxidizing activity, thereby enabling the analysis of L-glutamate. Other components can be used as they are in a configuration employed in well-known sensors, or modified as appropriate for use.

### EXAMPLES

The present invention is described below in detail with reference to Examples, but the present invention is not limited to the following Examples.

### [Example 1] Preparation of GluOX mutant

Each GluOX mutant was prepared as follows. First, a gene for GluOX (SEQ ID NO: 3) was chemically synthesized and cloned into a NdeI-HindIII cloning site of pET-16b (Merck & Co., Inc.). Hereinafter, a plasmid containing the GluOX sequence (SEQ ID NO: 3) with His-tag added to its N-terminus is referred to as pET-16b-GluOX. A reference GluOX (SEQ ID NO: 1) was also prepared in the same way. GluOX mutant was prepared using the KAPA HiFi HotStart ReadyMix PCR kit (NIPPON Genetics Co, Ltd.). The mutation was introduced into the GluOX gene using pET-16b-GluOX as a template according to a general protocol of site-directed mutagenesis. For all cases of single mutations of specific amino acids, insertions of specific amino acid residues and deletions of specific amino acid residues, mutated plasmids were prepared according to this method.

### [Example 2] Expression and purification of GluOX

A recombinant expression system for the reference GluOX and GluOX mutant was constructed using E. coli, and all were prepared by the same method. Herein, a method for expression and purification of GluOX of SEQ ID NO: 3 is described. Transformants of pET-16b-GluOX were obtained from E. coli BL21 (DE3) according to a standard method. Hereinafter, the transformant of BL21 (DE3) by pET-16b-GluOX is referred to as pET-16b-GluOX-BL21 (DE3).

The GluOX was prepared as follows. First, pET-16b-GluOX-BL21 (DE3) is transferred from a glycerol stock thereof onto an LB agar plate containing 100 ug/mL ampicillin for inoculation. Then, the plate was allowed to stand at 37°C overnight for incubation. Into a 14 mL round tube, 3 mL of LB liquid medium containing 100 ug/mL ampicillin was placed. A single colony in the LB plate was transferred into the medium for inoculation, and then incubated at 37°C overnight. Into a 50-mL tube, 6 mL of LB liquid medium containing 100 ug/mL ampicillin was placed. Then, 60 µL of culture solution was added to the medium, and incubated at 37°C by gyratory shaking until the OD₆₀₀ value reached approximately 0.6. The resulting culture solution was allowed to stand at 16°C for 30 minutes, supplemented with IPTG at a final concentration of 1.0 mM, and then incubated by gyratory shaking at 16°C overnight. Then, the bacterial cells were collected.

Bacterial cells were suspended in a disruption buffer (50 mM HEPES, 100 mM NaCl, pH 7.5) and then disrupted using an ultrasonic disruptor (BIORUPTOR, Cosmo Bio Co., Ltd.) in an amplitude mode of H for 10 min with 30-second intervals while cooling water was circulated. The sonicated solution was centrifuged at 13,000 × g for 15 min at 4°C. After collected, the supernatant was used for purification using a His SpinTrap (GE Healthcare Japan). Buffer used for equilibration and washing was (50 mM HEPES, 100 mM NaCl, pH 7.5). Elution buffer used was (50 mM HEPES, 100 mM NaCl, 500 mM imidazole, pH 7.5). The elution fractions were collected and diluted to 0.01 mg/mL with the elution buffer.

### [Example 3] Mutant screening by activity measurement

The activity was evaluated for the reference GluOX and GluOX mutant prepared in Examples 1 and 2 according to the following procedure. First, the absorbance at 555 nm was measured with a microplate reader (Varioskan LUX, Thermo Fisher Scientific, Inc.) for a mixture solution obtained by mixing 100 µL of 0.2 M HEPES, pH 7.5 with 20 µL of 10 mM glutamate solution, 20 µL of 30 mM N-ethyl-N-(2-hydroxy-3-sulfopropyl)-3-methoxyaniline solution (TOOS solution), 2 µL of 0.1 M 4-aminoantipyrine, 2 µL of 1500 U/mL peroxidase and 36 µL of ultrapure water. Then, 20 µL of GluOX solution prepared to 0.01 mg/mL was added, and then the change in the absorbance at 555 nm was measured over time with the microplate reader. Table 1 represents the activities of the reference GluOX and the GluOX mutant (SEQ ID NO: 3) lacking L467 to R506 and R670 to S687, in terms of relative activities with respect to that of the reference GluOX used as a control. Table 2 represents the activities of the reference GluOX and the GluOX mutants formed by inserting or modifying certain amino acid residues in the GluOX mutant (SEQ ID NO: 3) lacking L467 to R506 and R670 to S687, in terms of relative activities with respect to that of the reference GluOX used as a control. Table 3 represents the activities of the GluOX mutants (represented by a residue number in SEQ ID NO: 3) formed by modifying certain amino acid residues in the GluOX mutant (insertion mutant 6) that is formed by inserting GGGGS (SEQ ID NO: 4) between Y376 and A377 and inserting GGGGS (SEQ ID NO: 4) between E356 and L357 in the GluOX mutant lacking L467-R506 and R670-S687 in the GluOX mutant (SEQ ID NO: 3), in terms of relative activities with respect to that of the insertion mutant 6 used as a control.

**Table 1. Activities of reference GluOX and GluOX mutant lacking certain amino acid residues**

| ID | GluOX | Relative activity |
|---|---|---|
| Reference GluOX | Control | 1000 |
| Deletion mutant 1 | L467 to R506 and R670 to S687 deleted in SEQ ID NO: 1 (SEQ ID NO: 3) | 123% |

**Table 2. Activities of reference GluOX and GluOX mutants (represented by residue number in SEQ ID NO: 3) formed by inserting and/or modifying specific amino acid residues in GluOX mutant lacking L467 to R506 and R670 to S687 in reference GluOX (SEQ ID NO: 3)**

| ID | GluOX | Relative activity |
|---|---|---|
| Reference GluOX | Control | 100% |
| Mutant 1 | W313F | 120% |
| Mutant 2 | A106S | 118% |
| Mutant 3 | L505I | 158% |
| Mutant 4 | C210S | 119% |
| Mutant 5 | C561S | 104% |
| Inserted mutant 1 | GGGGS (SEQ ID NO: 4) inserted between Y376 and A377 | 146% |
| Inserted mutant 2 | GGGGS (SEQ ID NO: 4) inserted between E356 and L357 | 146% |
| Inserted mutant 3 | GGGGSGGGGS (SEQ ID NO: 5) inserted between E356 and L357 | 214% |
| Inserted mutant 4 | GGGGS (SEQ ID NO: 4) inserted between A466 and G467 | 104% |
| Inserted mutant 6 | P244H | 213% |
| Inserted mutant 5 | GGGGS (SEQ ID NO: 4) inserted between Y376 and A377, and P244H mutation | 338% |
| Inserted mutant 6 | GGGGS (SEQ ID NO: 4) inserted between Y376 and A377, and GGGGS (SEQ ID NO: 4) inserted between E356 and L357 | 489% |
| Inserted mutant 7 | GGGGS (SEQ ID NO: 4) inserted between Y376 and A377, and GGGGSGGGGS (SEQ ID NO: 5) inserted between A466 and G467 | 150% |
| Inserted mutant 8 | GGGGS (SEQ ID NO: 4) inserted between Y376 and A377, D461 to G468 deleted, and GGGGS (SEQ ID NO: 4) inserted between E460 and V469 | 316% |
| Inserted mutant 9 | GGGGS (SEQ ID NO: 4) inserted between Y376 and A377, and C210S mutation | 266% |
| Inserted mutant 10 | GGGGS (SEQ ID NO: 4) inserted between Y376 and A377, GGGGS (SEQ ID NO: 4) inserted between E356 and L357, and GGGGSGGGGS (SEQ ID NO: 5) inserted between A466 and G467 | 225% |
| Inserted mutant 11 | GGGGS (SEQ ID NO: 4) inserted between Y376 and A377, GGGGS (SEQ ID NO: 4) inserted between E356 and L357, D461 to G468 deleted, and GGGGS (SEQ ID NO: 4) inserted between E460 and V469 | 117% |

**Table 3. Activities of GluOX mutants (represented by residue number in SEQ ID NO: 3) formed by modifying certain amino acid residues in GluOX mutant (insertion mutant 6) that is formed by inserting GGGGS (SEQ ID NO: 4) between Y376 and A377 and inserting GGGGS (SEQ ID NO: 4) between E356 and L357 in SEQ ID NO: 3**

| ID | GluOX | Relative activity |
|---|---|---|
| Inserted mutant 6 | GGGGS (SEQ ID NO: 4) inserted between Y376 and A377 and GGGGS (SEQ ID NO: 4) inserted between E356 and L357 | 100% |
| Mutant 7 | Q235E | 116% |
| Mutant 8 | D236E | 177% |
| Mutant 9 | D237E | 133% |
| Mutant 10 | Q333E | 157% |
| Mutant 11 | I334V | 158% |
| Mutant 12 | I334L | 187% |
| Mutant 13 | M336L | 111% |
| Mutant 14 | Q338E | 161% |
| Mutant 15 | A438P | 103% |
| Mutant 16 | K441E | 108% |
| Mutant 17 | Y455F | 127% |
| Mutant 18 | Q456R | 120% |
| Mutant 19 | Q457E | 156% |
| Mutant 20 | Q457K | 132% |
| Mutant 21 | P558A | 101% |
| Mutant 22 | P569A | 1460% |
| Mutant 23 | P244H | 103% |
| Mutant 24 | D236E and Q338E | 231% |
| Mutant 25 | D236E and R339K | 1540% |
| Mutant 26 | D236E and T416S | 290% |
| Mutant 27 | T311S and D236E | 303% |
| Mutant 28 | T311S and Q457E | 184% |
| Mutant 29 | Q457E and D236E | 176% |
| Mutant 30 | Q457E and T311S | 166% |
| Mutant 31 | Q457E and Q338E | 104% |
| Mutant 32 | Q457E and R339K | 133% |

### [Example 4] Determination of boundary proximity region

3D structure (PDB ID: 2E1M) of a hetero octamer (α1₂α2₂β₂γ₂) of naturally-occurring GluOX was not observed partially when displayed by PyMOL, a molecular graphics software. It results from uncertain 3D structure that is obtained due to high mobility of the region when experimentally determined by X-ray crystallography for protein 3D structure. Table 4 represents regions of the amino acid residues in SEQ ID NO: 3 where 3D structure was confirmed for the hetero-octamer of naturally-occurring GluOX (α1₂α2₂β₂γ₂) . Based on the results, the boundary proximity regions were determined to include the region where the 3D structure was not observed. In other words, the boundary proximity regions in SEQ ID NO: 3 correspond to the regions as follows. A region in proximity to a boundary between α1 and α2 regions: a region consisting of 349th to 363rd amino acid residues; a region in proximity to a boundary between α2 and γ regions: a region consisting of 372nd to 377th amino acid residues; and a region in proximity to a boundary between γ and β regions: a region consisting of 466th to 469th amino acid residues.

**Table 4. Regions of amino acid residues in SEQ ID NO: 3 where 3D structure was confirmed for hetero-octamer of naturally-occurring GluOX (α1₂α2₂β₂γ₂)**

| Amino acid residues in SEQ ID NO: 3 | Whether or not structure of amino acid residue is observable |
|---|---|
| Positions 1 to 349 | ○ |
| Positions 350 to 362 | × |
| Positions 363 to 372 | ○ |
| Positions 373 to 376 | × |
| Positions 377 to 466 | ○ |
| Positions 467 to 468 | × |
| Positions 469 to 619 | ○ |
| Positions 620 to 629 | × |

| | |
|---|---|
| ∘: Yes (observable) ×: No (not observable) | |

As described above, the L-glutamate oxidase mutant of the present invention has an improved enzyme activity compared to that of wild type, and is therefore useful for fast and highly sensitive measurement of L-glutamate and/or for production of 2-oxoglutarate and/or for use as a testing reagent for L-glutamate.

### Industrial Applicability

This invention is useful in a wide range of fields, including biological research, health nutrition, medication and food production.

[SEQUENCE LISTING]

## Claims

1. An L-glutamate oxidase mutant of (a) or (b):
(a) an L-glutamate oxidase mutant comprising an amino acid sequence that has 90% or more identity to the amino acid sequence of SEQ ID NO: 3 and exhibits an activity of oxidizing L-glutamate,
except an L-glutamate oxidase comprising the amino acid sequence of SEQ ID NO: 1; or
(b) an L-glutamate oxidase mutant comprising a peptide linker consisting of 1 to 20 amino acid residues which is inserted into one or more sites selected from the group consisting of (1) a site in a region in proximity to a boundary between α1 and α2 regions, (2) a site in a region in proximity to a boundary between α2 and γ regions and (3) a site in a region in proximity to a boundary between γ and β regions in the L-glutamate oxidase mutant (a), and having the activity of oxidizing L-glutamate,
wherein the site in the region in proximity to the boundary between α1 and α2 regions is a site in a region consisting of 349th to 363rd amino acid residues of SEQ ID NO: 3,
the site in the region in proximity to the boundary between α2 and γ regions is a site in a region consisting of 372nd to 377th amino acid residues of SEQ ID NO: 3, and
the site in the region in proximity to the boundary between γ and β regions is a site in a region consisting of 466th to 469th amino acid residues of SEQ ID NO: 3.

2. The L-glutamate oxidase mutant according to claim 1, wherein the L-glutamate oxidase mutant is a mutant of an L-glutamate oxidase derived from a microorganism belonging to the genus *Streptomyces.*

3. The L-glutamate oxidase mutant according to claim 2, wherein the microorganism belonging to the genus *Streptomyces* is *Streptomyces sp.* X-119-6.

4. The L-glutamate oxidase mutant according to any one of claims 1 to 3, wherein the peptide linker is inserted into either one or both of (1) the site in the region in proximity to the boundary between α1 and α2 regions, or (2) the site in the region in proximity to the boundary between α2 and γ regions.

5. The L-glutamate oxidase mutant according to any one of claims 1 to 4,
wherein the site in the region in proximity to the boundary between α1 and α2 regions is a site between 356th and 357th amino acid residues in SEQ ID NO: 3,
the site in the region in proximity to the boundary between α2 and γ regions is a site between 376th and 377th amino acid residues in SEQ ID NO: 3, or
the site in the region in proximity to the boundary between γ and β regions is a site between 466th and 467th amino acid residues in SEQ ID NO: 3.

6. The L-glutamate oxidase mutant according to any one of claims 1 to 5, comprising a mutation or mutations of one or more amino acid residues selected from the group consisting of A106, C210, Q235, D236, D237, P244, T311, W313, Q333, 1334, M336, Q338, R339, T416, A438, K441, Y455, Q456, Q457, L505, P558, C561 and P569 in SEQ ID NO: 3.

7. The L-glutamate oxidase mutant according to any one of claims 1 to 6, comprising a mutation or mutations of one or more amino acid residues selected from the group consisting of A106S, C210S, Q235E, D236E, D237E, P244H, T311S, W313F, Q333E, I334V, I334L, M336L, Q338E, R339K, T416S, A438P, K441E, Y455F, Q456R, Q457E, Q457K, L505I, P558A, C561S and P569A in SEQ ID NO: 3.

8. A method of analyzing L-glutamate, the method comprising measuring L-glutamate contained in a test sample using the L-glutamate oxidase mutant according to any one of claims 1 to 7.

9. The method according to claim 8, wherein the L-glutamate is measured using N-ethyl-N-(2-hydroxy-3-sulfopropyl)-3-methoxyaniline (TOOS) and 4-aminoantipyrine and a peroxidase in addition to the L-glutamate oxidase mutant according to any one of claims 1 to 7.

10. A method for producing 2-oxoglutaric acid, the method comprising producing 2-oxoglutaric acid from L-glutamate in presence of the L-glutamate oxidase mutant according to any one of claims 1 to 7.

11. A polynucleotide encoding the L-glutamate oxidase mutant according to any one of claims 1 to 7.

12. An expression vector comprising the polynucleotide according to claim 11.

13. A transformed microorganism comprising an expression unit containing a polynucleotide encoding the L-glutamate oxidase mutant according to any one of claims 1 to 7 and a promoter operably linked to the polynucleotide.

14. A method of producing the L-glutamate oxidase mutant according to any one of claims 1 to 7, comprising producing the L-glutamate oxidase mutant using the transformed microorganism according to claim 13.

15. An L-glutamate detection reagent or kit comprising the L-glutamate oxidase mutant according to any one of claims 1 to 7.

16. The L-glutamate detection reagent or kit according to claim 15, further comprising one or more selected from the group consisting of a buffer solution or buffer salt for reaction, a hydrogen peroxide detection reagent, an ammonia detection reagent, and a 2-oxoglutaric acid detection reagent.

17. A detection system for analysis of L-glutamate, the detection system comprising:
(a) a device; and
(b) the L-glutamate oxidase mutant according to any one of claims 1 to 7.

18. The detection system for analysis of L-glutamate according to claim 17, the system further comprising (c) one or more selected from the group consisting of a buffer solution or buffer salt for reaction, a hydrogen peroxide detection reagent, an ammonia detection reagent, and a 2-oxoglutaric acid detection reagent.

19. An enzyme sensor for analysis of L-glutamate, the enzyme sensor comprising:
(a) an electrode for detection; and
(b) the L-glutamate oxidase mutant according to any one of claims 1 to 7 that is immobilized or retained on the electrode for detection.
